# FASCICULE DE BREVET EUROPEEN

(11) **EP 3 773 282 B1**
(45) Date de publication et mention de la délivrance du brevet: **02.04.2025**
(21) Numéro de dépôt: 19723152.5
(22) Date de dépôt: 12.04.2019
(51) Int. Cl.: A61B 17/70

(54) **ENSEMBLE DE TRIANGULATION PERMETTANT LE REDRESSEMENT DE VERTÈBRES, ET SYSTÈME D'OSTEOSYNTHÈSE RACHIDIENNE COMPRENANT DE TELS ENSEMBLES**
TRIANGULATIONSANORDNUNG ZUM KORRIGIEREN DER WIRBELSÄULE UND WIRBELSÄULENOSTEOSYNTHESESYSTEM MIT SOLCHEN ANORDNUNGEN
TRIANGULATION ASSEMBLY FOR RECTIFYING VERTEBRAE, AND SPINAL OSTEOSYNTHESIS SYSTEM COMPRISING SUCH ASSEMBLIES

(30) Priorité: 12.04.2018 FR 1853204
(43) Date de publication de la demande: 17.02.2021
(73) Titulaire: Stryker European Operations Limited, Carrigtwohill, Co. Cork T45 HX08 (IE)
(72) Inventeur: ASSAKER, Richard, 7540 Kain (BE); CHOPIN, Daniel, 71640 Mercurey (FR); PETIT, Dominique, 62180 Verton (FR)
(74) Mandataire: Regimbeau
(86) Numéro de dépôt international: PCT/FR2019/050866
(87) Numéro de publication internationale: WO 2019/197787

(56) Documents cités:
- WO-A1-01/54597
- FR-A1- 2 761 876
- US-A- 5 196 013
- US-A- 5 425 732
- US-A1- 2010 211 100
- US-A1- 2016 015 427
- US-B1- 6 749 613
- US-B1- 9 198 696
- US-B2- 7 794 464
- US-B2- 9 211 145

## Description

### DOMAINE TECHNIQUE DE L'INVENTION

L'invention concerne un ensemble permettant le redressement de vertèbres, et notamment mais non exclusivement de vertèbres lombaires par voie postérieure, et un système d'ostéosynthèse rachidienne comprenant de tels ensembles.

### ETAT DE LA TECHNIQUE

De manière conventionnelle, il est procédé au redressement des vertèbres lombaires au moyen de systèmes d'ostéosynthèse rachidienne comportant des moyens d'ancrage osseux, du type vis pédiculaires, implantés dans des vertèbres successives concernées par le redressement, et reliés entre eux longitudinalement par des tiges ou plaques de liaison par l'intermédiaire de moyens de connexion. Par exemple, le document US 9 198 696 B1 divulgue des connecteurs croisés utilisés pour relier des structures bilatérales de fixation de la colonne vertébrale, et le document US 9 211 145 B2 divulgue un procédé et un système de traitement de déformations de la colonne vertébrale.

Le redressement des vertèbres via les systèmes d'ostéosynthèse rachidienne lombaire existants rencontre cependant plusieurs problématiques liées notamment au cintrage et à la mise en place des tiges de liaison.

Dans le cas des vis pédiculaires monoaxiales, il est en effet nécessaire de procéder à des adaptations des tiges de liaison par des courbures adaptées. Or le cintrage des tiges de liaison reste une opération peu évidente et peu précise, celui-ci étant d'autant plus difficile que la déformation de la colonne vertébrale est importante. En outre, la correction est de fait limitée par la courbure donnée à la tige de liaison en combinaison avec l'angle d'insertion des vis dans les pédicules. Par ailleurs, en cas de forte correction, les efforts de correction qui sont directement appliqués sur les vis pédiculaires peuvent affecter la qualité de l'ancrage, et en particulier dans le cas d'os ostéoporotique, provoquer un arrachement des vis pédiculaires.

Afin de s'affranchir de ces inconvénients des vis pédiculaires monoaxiales, il est connu de remplacer les vis monoaxiales par des vis pédiculaires multiaxiales. L'utilisation des vis multiaxiales ne s'avère cependant pas entièrement satisfaisante car elles requièrent de procéder à la réduction des vertèbres soit par la technique de mise en position du patient, soit par des techniques de cintrage de la tige de liaison, techniques se révélant cependant relativement lourdes, longues et peu aisées et n'assurant pas systématiquement, comme les vis pédiculaires monoaxiales, un redressement correct des vertèbres. En outre l'utilisation de vis multiaxiale a pour contrepartie une perte de correction.

Ainsi, que les vis pédiculaires utilisées soient des vis monoaxiales ou multiaxiales, il s'avère particulièrement difficile de parvenir à une correction dans les 3 dimensions (sagittale, coronale, et transverse) précise. Il ne peut en outre être assuré systématiquement un redressement correct des vertèbres.

L'invention vise à remédier à ces problèmes en proposant un ensemble de redressement, ainsi qu'un système d'ostéosynthèse rachidienne comprenant de tels ensembles, permettant des corrections sagittale, coronale, et transverse précises avec une capacité importante pour ces corrections tout en assurant une qualité d'accroche sur les vertèbres satisfaisante.

### OBJET DE L'INVENTION

A cet effet, et selon un premier aspect, l'invention concerne un ensemble de redressement de vertèbres comprenant deux moyens d'ancrage osseux destinés à être implantés sur une même vertèbre, une structure de pontage des moyens d'ancrage osseux entre eux, ladite structure de pontage, implantable, comprenant une barre de triangulation et des moyens de connexion permettant de raccorder la barre de triangulation à chacun des moyens d'ancrage osseux, un instrument de correction monté sur la barre de triangulation, des moyens de blocage de la barre de triangulation sur les moyens d'ancrage osseux par l'intermédiaire des moyens de connexion, la barre de triangulation formant ainsi avec les moyens d'ancrage osseux un ensemble rigide de forme triangulaire, et des moyens de réglage de la position angulaire de l'instrument de correction par rapport à la vertèbre selon un angle déterminé.

La barre de triangulation implantée, associée à la position convergente des moyens d'ancrage osseux inhérente à la constitution même de la vertèbre, permet d'obtenir un ensemble de fixation triangulaire rigide, renforcé et indissociable des vertèbres. Cela permet ainsi de procéder à des manœuvres de correction dans les 3 dimensions directement appliquées sur la vertèbre sur laquelle l'ensemble est monté, et ce sans risque d'endommager les ancrages osseux. Par ailleurs, la possibilité de régler la position angulaire de l'instrument de correction par rapport à la vertèbre sur laquelle il est monté va permettre au chirurgien d'établir un référentiel permettant de parvenir de manière simple et aisée au redressement souhaité de la vertèbre.

Selon une première configuration de l'invention, les moyens de connexion comportent les moyens de réglage de la position angulaire de l'instrument de correction.

Avantageusement, la barre de triangulation comporte une zone d'accroche de l'instrument de correction arrangée pour assurer une fixation solidaire en rotation et en translation de l'instrument de correction sur la barre de triangulation.

Avantageusement, la barre de triangulation comporte un méplat formant la zone de fixation.

Selon une autre configuration, les moyens de réglage de la position angulaire de l'instrument de correction sont ménagés sur la barre de triangulation.

Dans un exemple , la barre de triangulation comporte une zone d'accroche de l'instrument de correction arrangée pour autoriser au moins un débattement de l'instrument de correction sur la barre de triangulation dans le plan sagittal, la zone d'accroche constituant les moyens de réglage de la position angulaire de l'instrument de correction. Selon un mode de réalisation particulier, la barre de triangulation comporte une partie crantée formant la zone d'accroche.

Avantageusement, la zone d'accroche est centrale.

Les moyens de connexion comprennent deux connecteurs comprenant chacun une première zone de passage définissant un axe longitudinal de passage pour la barre de triangulation et une deuxième zone de passage arrangée pour recevoir l'un des moyens d'ancrage osseux, ladite deuxième zone de passage présentant une forme oblongue orientée parallèlement à l'axe longitudinal de passage de la première zone de passage, les connecteurs constituant les moyens de réglage de la position angulaire de l'instrument de correction.

Avantageusement, la deuxième zone de passage est décalée par rapport à l'axe longitudinal de passage.

Avantageusement, la première zone de passage est conformée pour permettre le coulissement et la rotation de la barre de triangulation en son sein avant son blocage sur les moyens d'ancrage osseux. Après blocage de la barre de triangulation sur les deux moyens d'ancrage osseux ces derniers forment, avec la barre de triangulation un ensemble de fixation triangulaire rigide dépourvu de degré de liberté.

Selon une configuration particulière, chaque connecteur se présente sous la forme d'une pince comprenant une partie ayant une section en C prolongée par deux bras comportant respectivement un orifice, les orifices de chaque bras étant disposés en vis-à-vis l'un de l'autre de sorte à définir la deuxième zone de passage tandis que la section en C délimite la première zone de passage. Avantageusement, il peut être prévu que les orifices de bras soient arrangés pour présenter un décalage axial l'un par rapport à l'autre. Cet arrangement permet d'obtenir un serrage sur la tige plus important du fait de l'effet de cisaillement.

Avantageusement, chaque moyen d'ancrage osseux comprend une tête de vis fixe prolongée de part et d'autre par une partie filetée inférieure pour l'implantation dans la vertèbre et par une partie filetée supérieure sur laquelle un écrou de serrage formant les moyens de blocage est destiné à être vissé.

Avantageusement, la barre de triangulation est droite ou cintrée.

L'invention concerne également un connecteur destiné à être mis en oeuvre avec un ensemble de redressement comme par exemple celui décrit précédemment, le connecteur étant arrangé pour permettre le réglage de la position angulaire d'un instrument de correction. Le connecteur reprend l'ensemble des caractéristiques décrites précédemment. En particulier, il comporte une première zone de passage définissant un axe longitudinal de passage d'une barre de triangulation et une deuxième zone de passage arrangée pour recevoir un moyen d'ancrage osseux, laquelle deuxième zone de passage présente une forme oblongue orientée parallèlement à l'axe longitudinal de passage de la première zone de passage. Selon une configuration particulière, le connecteur se présente sous la forme d'une pince comprenant une partie ayant une section en C prolongée par deux bras comportant respectivement un orifice, les orifices de chaque bras étant disposés en vis-à-vis l'un de l'autre de sorte à définir la deuxième zone de passage tandis que la section en C délimite la première zone de passage. Avantageusement, il peut être prévu que les orifices de bras soient arrangés pour présenter un décalage axial l'un par rapport à l'autre. Cet arrangement permet d'obtenir un serrage sur la tige plus important du fait de l'effet de cisaillement.

L'invention concerne également un système d'ostéosynthèse rachidienne comprenant au moins deux ensembles de redressement de vertèbres tels que décrits précédemment, lesdits ensembles étant destinés à être implantés sur deux vertèbres distinctes et à être reliés entre eux.

Avantageusement, le système comporte au moins un instrument de manœuvre reliant les instruments de correction de chacun des ensembles de triangulation entre eux.

Il est également décrit un procédé, non compris dans la portée des revendications, pour redresser des vertèbres à l'aide de deux ensembles de redressement de vertèbres tels que décrits précédemment et comprenant les étapes suivantes :
- implanter des premiers moyens d'ancrage osseux dans une première vertèbre,
- implanter des deuxièmes moyens d'ancrage osseux dans une deuxième vertèbre
- relier les premiers moyens d'ancrage osseux entre eux au moyen d'une première barre de triangulation comprenant des premier moyens d'accroche d'un premier instrument de correction, ladite première barre de triangulation formant une première structure de pontage implantée,
- relier les deuxièmes moyens d'ancrage osseux entre eux au moyen d'une deuxième barre de triangulation comprenant des deuxièmes moyens d'accroche d'un deuxième instrument de correction, ladite deuxième barre de triangulation formant une première structure de pontage implantée,
- positionner les premiers et deuxièmes instruments de correction l'un par rapport à l'autre selon un angle correspondant à la correction angulaire sagittale souhaitée et bloquer les premier et deuxième instruments de correction en position,
- et, après blocage des premier et deuxième instruments de correction en position, déplacer le premier et/ou le deuxième instrument de correction pour les positionner parallèles entre eux.

Selon un premier mode de réalisation non compris dans la portée des revendications, le positionnement des premier et deuxième instruments de correction selon un angle correspondant à la correction angulaire sagittale souhaitée est réalisé par pivotement des première et deuxième barre de triangulation sur les premier et deuxième moyens d'ancrage osseux. Le réglage de la position angulaire des instrument de correction est rendu possible par la mise en œuvre de moyens de connexion des barres de triangulation sur les moyens d'ancrage osseux, lesquels moyens de connexion sont arrangés pour autoriser, avant blocage, un mouvement multiaxial des barres de triangulation sur les moyens d'ancrage osseux. Le blocage des premier et deuxième instruments de correction en position est alors réalisé par blocage des barres de triangulation sur les premier et deuxième moyens d'ancrage osseux.

Selon un autre mode de réalisation non compris dans la portée des revendications, le positionnement des premier et deuxième instruments de correction selon un angle correspondant à la correction angulaire sagittale souhaitée est réalisé par pivotement des instruments de correction sur leur barres de triangulation respectives via les moyens d'accroche desdits barres arrangés pour permettre un tel mouvement de pivotement.

La mise en place des barres de triangulation et le positionnement des instruments de correction selon un angle défini avant blocage permet un contrôle optimal et précis des corrections sagittale, coronale et transverse.

Avantageusement, les premier et deuxième instruments de correction sont fixés respectivement sur la première et deuxième barre de triangulation, au niveau des premier et deuxième moyens d'accroche, préalablement à la mise en place desdites barres de triangulation sur les moyens d'ancrage osseux associés.

### BREVE DESCRIPTION DES FIGURES

D'autres objets et avantages de l'invention apparaîtront au cours de la description qui suit, faite en référence aux dessins annexés, dans lesquels :
- la figure 1 représente une vue schématique d'un ensemble de redressement de vertèbres selon un exemple de réalisation ;
- la figure 2 représente une vue éclatée partielle de l'ensemble de redressement de vertèbres de la figure 1 ;
- la figure 3 représente une vue de dessus de la barre de triangulation mise en œuvre dans l'ensemble de redressement de vertèbres de la figure 1 ;
- la figure 4 représente un autre exemple de réalisation de la barre de triangulation ;
- les figures 5 à 7 représentent des vues respectivement en perspective, de dessus et de côté du connecteur mis en œuvre dans l'ensemble de redressement de vertèbre de la figure 1 ;
- les figures 8 et 9 représentent une vue de détail en perspective de l'ensemble de redressement, respectivement avant et après la mise en place de l'écrou de serrage ;
- les figures 10 et 11 représentent une vue latérale de l'ensemble de redressement illustré sur les figures 8 et 10 ;
- les figures 12 à 18b représentent les étapes de mise en place de deux ensembles de redressement en vue du redressement de vertèbres ;
- les figures 19a à 19c montrent des vues respectivement en perspective, transversale et postérieure de vertèbres nécessitant un redressement dans les 3 dimensions, lesdites vertèbres étant équipées d'ensembles de redressement selon l'invention ;
- les figures 20a et 20b montrent les vertèbres illustrées sur les figures 19a à 19c après une correction de rotation transverse ;
- les figures 21a à 21c montrent les vertèbres illustrées sur les figures 20a et 20b après une correction du déplacement coronal ;
- la figure 22 représente une vue schématique d'un ensemble de redressement de vertèbres selon un autre exemple de réalisation ;
- la figure 23 représente une vue éclatée partielle de l'ensemble de redressement de vertèbres de la figure 22.

### DESCRIPTION DETAILLEE DES FIGURES

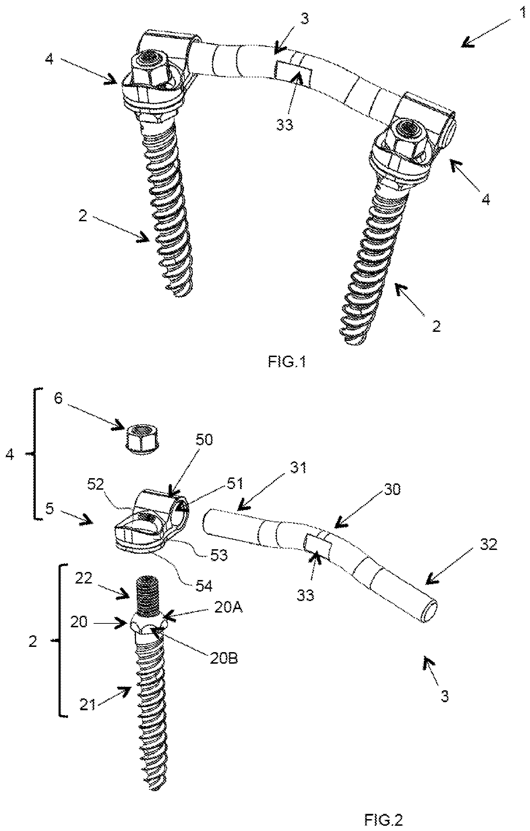

En relation avec les figures 1 à 11, il est décrit un exemple de réalisation d'un ensemble de redressement 1 de vertèbres selon l'invention.

L'ensemble de redressement 1 comporte deux moyens d'ancrage osseux 2 destinés à être implantés sur une même vertèbre 100, une barre de triangulation 3 et des moyens de connexion 4 permettant de raccorder la barre de triangulation à chaque moyen d'ancrage osseux.

Dans le mode de réalisation illustré, la barre de triangulation 3 (de section circulaire dans l'exemple) présente avantageusement une forme en Oméga afin de suivre la forme de la vertèbre 100 sur laquelle elle est montée. Plus particulièrement, elle comporte une partie centrale 30 et deux parties d'extrémité 31, 32 respectivement linéaires, lesdites parties d'extrémité étant reliées chacune à la partie centrale par une portion courbe.

Afin de procéder au redressement des vertèbres selon la technique décrite plus loin, la barre de triangulation 3 comporte une zone d'accroche 33 permettant l'accroche d'un instrument de correction 10. Dans le mode de réalisation illustré, la zone de d'accroche 33 est prévue au niveau de la partie centrale 30. Elle est formée de deux méplats disposés de part et d'autre de la barre. Ils sont ménagés de manière centrale dans la partie cintrée.

Les moyens de connexion 4 associés à chaque moyen d'ancrage 2 comportent un connecteur 5 et un écrou de serrage 6.

Chaque connecteur 5 comportant une première et une deuxième zone de passage 51, 52, la première zone étant arrangée pour recevoir les parties d'extrémité 31, 32 de la barre de triangulation 3, la deuxième zone étant arrangée pour recevoir l'un des moyens d'ancrage osseux 2. Avantageusement, la deuxième zone de passage 52 est décalée par rapport à la première zone de passage 51 et définit un axe longitudinal de passage perpendiculaire à l'axe longitudinal de passage de la première zone de passage 51.

Selon une configuration avantageuse, la deuxième zone de passage 52 est avantageusement de forme oblongue pour permettre la fixation de l'ensemble de redressement dans une configuration où la barre de triangulation 3 et le moyen d'ancrage 2 ne seraient pas perpendiculaires. Dans le mode de réalisation décrit, le débattement angulaire est de +/- 15°. Avantageusement, la deuxième zone de passage 52 est arrangée pour permettre une rotation du connecteur 5 sur le moyen d'ancrage 2, et ainsi permettre l'adaptabilité de la barre de triangulation lorsque celle-ci n'est pas perpendiculaire aux moyens d'ancrage 2.

Dans le mode de réalisation illustré, chaque connecteur 5 se présente sous la forme d'une pince, de forme générale en U, comprenant deux branches 53, 54 s'étendant en regard et à distance l'une de l'autre et, au niveau de la naissance des branches 53, 54, une portion tubulaire 50 de section en C définissant la première zone de passage 51 pour recevoir la barre de triangulation 3. Chaque branche 53, 54 du connecteur comporte un orifice traversant 523, 524 définissant la deuxième zone de passage 52. Avantageusement, les orifices traversant 523, 524 présentent respectivement une forme oblongue orientée parallèlement à l'axe longitudinal de passage de la première zone de passage 51, l'orifice traversant 523 présentant une longueur supérieure à celle de l'orifice traversant 524. Cet arrangement des orifices de forme oblongues 523, 524 a pour avantage de permettre la libre rotation du connecteur 5 sur le moyen d'ancrage osseux avant son blocage. Comme illustré sur les figures, la deuxième zone de passage 52 est décalée de l'axe longitudinal de passage.

Dans le mode de réalisation illustré, les orifices traversant 523, 524 sont disposés coaxialement, en regard l'un de l'autre. Selon une variante de réalisation, les orifices traversant 523, 524 peuvent être arrangés pour présenter un léger décalage l'un par rapport à l'autre afin d'assurer une pression de serrage sur la tige plus importante par effet de cisaillement.

Avantageusement, l'orifice traversant 523 de la branche supérieure (branche 53) est délimité par une surface d'appui interne 523A présentant une forme incurvée conformée pour recevoir un des écrous de serrage 6, lesdits écrous de serrage 6 présentant chacun une embase de forme convexe apte à coopérer avec la surface d'appui interne de l'orifice traversant 523 de la branche 53.

Avantageusement, la première zone de passage 51 est conformée pour permettre le coulissement et la rotation de la barre de triangulation 3 en son sein avant son blocage sur les connecteurs 5 via les écrous de serrage 6.

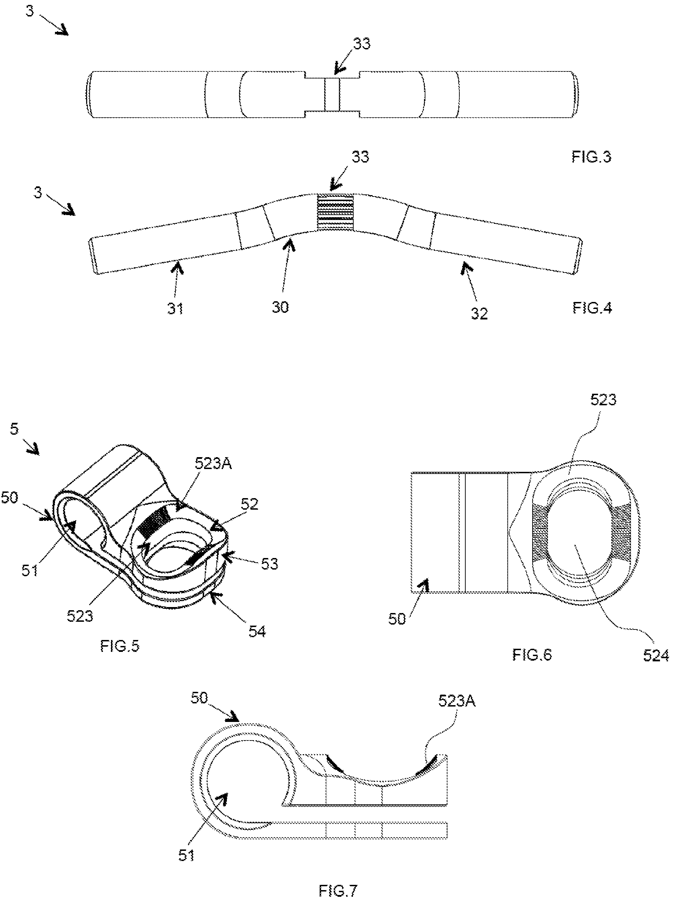

Les connecteurs venant d'être décrits sont ainsi arrangés pour former des moyens de réglage qui vont permettre de régler la position angulaire d'un instrument de correction monté sur la barre de triangulation 3 comme cela sera décrit plus loin. Il peut être prévu également que les moyens de réglage soient portés, non pas par les connecteurs, mais par la barre de triangulation 3. La figure 4 donne un exemple de barre de triangulation 3 dans laquelle la zone d'accroche 33 comporte un crantage. Il est ainsi possible de régler la position angulaire de l'instrument de correction.

Dans le mode de réalisation illustré, les moyens d'ancrage osseux 2 se présentent sous la forme d'une vis d'ancrage à tête de pans. Dans ce qui suit, on parlera indifféremment de « moyens d'ancrage osseux » ou de « vis d'ancrage ».

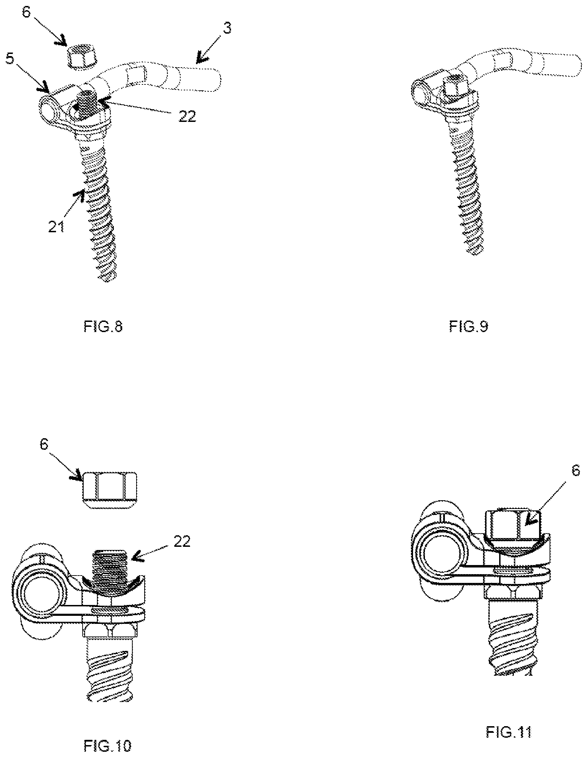

Chaque vis d'ancrage 2 comprend une tête de vis 20 prolongée de part et d'autre par des parties filetées 21, 22, la partie filetée 21 inférieure étant conformée pour l'implantation dans la vertèbre, la partie filetée 22 supérieure étant conformée pour recevoir un des écrous de serrage 6. Par ailleurs, la tête de vis 20 comporte une face supérieure 20A de forme sphérique convexe et, en partie inférieure, une face latérale 20B présentant une empreinte à 6 pans. Comme on le verra plus loin, le connecteur 5 est positionné sur la vis d'ancrage 2 associée, la face inférieure de la branche inférieure 54, de forme avantageusement complémentaire, venant en appui contre la face supérieure 20A de forme sphérique de la tête de vis 20, cette dernière jouant un rôle de pivot lors du redressement des vertèbres. L'écrou de serrage 6, sous l'application d'un couple de serrage, provoque le rapprochement des branches 50, 51 l'une par rapport à l'autre, jusqu'au blocage de la barre de triangulation 3 dans le connecteur 4 (figures 8 à 11).

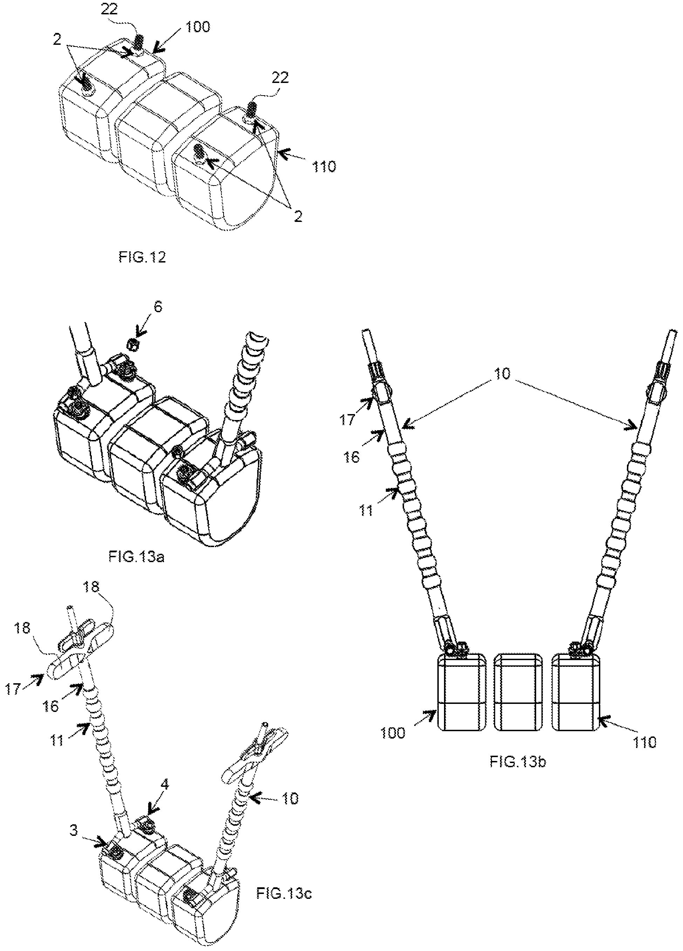

Les figures 12 à 19b illustrent la mise en place de deux ensembles de redressement 1 tels que décrits précédemment et leur mise en œuvre pour le redressement de vertèbres.

On procède tout d'abord à la mise en place des vis d'ancrage 2 de chacun des ensembles de redressement sur les deux vertèbres 100, 110 situées de part et d'autre de la zone à traiter de la colonne vertébrale (figure 12). Chaque vis d'ancrage 2 est implantée au niveau d'un des pédicules de la vertèbre associée (les figures montrent des vertèbres schématisées). Les vis d'ancrage 2 de chaque vertèbre 100, 110 sont implantées de manière à converger l'une en direction de l'autre.

On relie ensuite les vis d'ancrage 2 d'une même vertèbre entre elles avec une barre de triangulation 3 en enfilant, au travers du deuxième passage 52 de chaque connecteur 5 équipant les extrémités de ladite barre, la partie filetée 22 supérieure de la vis d'ancrage 2 concernée jusqu'à ce que la branche inférieure 54 du connecteur 5 vienne en appui sur la face supérieure sphérique 20A de la tête de vis 20. Cette étape est réalisée à l'aide d'un instrument de manipulation 10 préalablement fixé sur la zone d'accroche de la barre de triangulation 3 (figures 13a et 13b). Dans le mode de réalisation illustré, l'instrument de manipulation 10 est destiné à assurer également la correction des vertèbres. On le désignera par la suite instrument de correction 10. Dans le mode de réalisation illustré, l'instrument de correction 10 est formé d'un axe 16 pourvu d'une pluralité de moyens d'accroche 11 destinés à permettre l'accroche d'instruments additionnels, et notamment d'instruments dits de manœuvre. Avantageusement, les moyens d'accroche 11 forment une liaison rotule pour permettre une connexion multi-directionnelle avec les instruments additionnels, ladite liaison rotule pouvant être bloquée à tout moment. L'instrument de correction 10 comporte, au niveau de l'extrémité opposée à l'extrémité d'accroche sur la barre de triangulation 3, une poignée 17. Avantageusement, la poignée 17 est arrangée pour présenter des extensions 18 s'étendant de part et d'autre de l'axe, et avantageusement perpendiculaires à celui-ci, dans le plan transverse des vertèbres lorsque l'instrument de correction 10 est monté sur celui-ci.

Les écrous de serrage 6 sont ensuite placés sur la partie filetée 22 supérieure de la tête de vis 20 émergeant du connecteur 5 sans toutefois être vissés complètement afin de ne pas bloquer d'une part le déplacement de la barre de triangulation 3 au travers du connecteur 5 et d'autre part le mouvement des connecteurs 5 sur les vis d'ancrage 2 (figure 13c).

Les instruments de correction 10 sont ensuite positionnés l'un par rapport à l'autre selon un angle α correspondant à la correction angulaire sagittale prédéterminée par le chirurgien, lequel est choisi généralement pour obtenir un équilibre sagittal optimum du patient à l'aide d'un instrument de mesure d'angle 15. Dans le cas d'une ostéotomie, le positionnement angulaire des instruments de correction 10 est choisi pour correspondre à l'angle de lordose recherchée au final.

Une fois l'angle entre les instruments de correction 10 atteint, il est procédé au serrage final des écrous 6 pour maintenir les ensembles de fixation triangulaires dans une position fixe et rigide sur chacune des vertèbres.

Des instruments de manœuvre 12, 13 reliant les instruments de correction 10 de chacun des ensembles de triangulation, sont ensuite mis en place. sur les moyens d'accroche 11 des instruments de correction 10. Les instruments de manœuvre comprennent un système à crémaillère, permettant un écartement ou rapprochement progressif des instruments de correction 10 entre eux via les ensembles de triangulation, et donc une compression ou distraction progressives des vertèbres entre elles. La position fixe et rigide de l'ensemble de fixation obtenue à l'aide des instruments de manœuvre permet de stabiliser les vertèbres dans n'importe quelles positions, et ainsi de réaliser nombre de gestes chirurgicaux sur une colonne vertébrale stable. Dans le cas d'une ostéotomie, cette position fixe et rigide permet une stabilité essentielle au geste chirurgical, l'ablation partielle d'un corps vertébral ayant pour conséquence une instabilité importante de la colonne vertébrale.

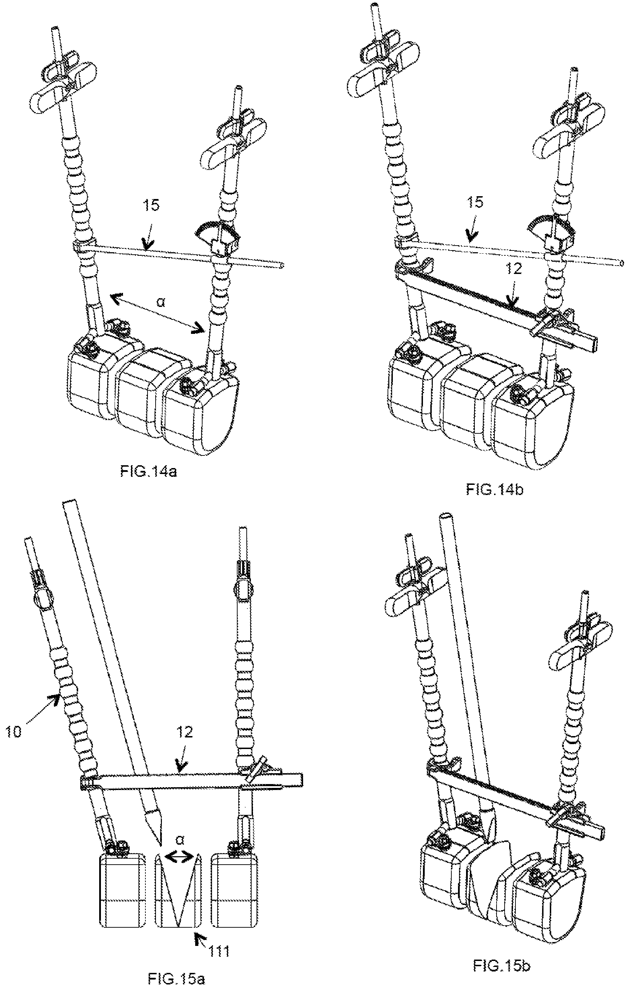

Un premier instrument de manœuvre 12, reliant les instruments de correction 10 de chacun des ensembles de redressement, est ainsi mis en place (figure 14a et 14b). Ce premier instrument permet, outre la compression/distraction des vertèbres 100, 110 réalisée ultérieurement, une correction angulaire des vertèbres. Le déplacement antéro-postérieur de cet instrument de manœuvre, permet également une correction directe de positionnement antéro-postérieure des vertèbres, avantageusement dans le cas de spondylolisthésis. Ce déplacement/correction est possible par la combinaison des instruments de correction 10, instrument de manœuvre positionné en forme de cadre et du moyen d'accroche entre ces instruments de type rotule. Une poignée non représentée ici, pourrait également être positionnée en extrémité de l'instrument de manœuvre afin de faciliter ce déplacement. Ce premier instrument a également pour avantage d'éviter toute instabilité du rachis lorsque les rotules sont bloquées.

Lorsqu'une ostéotomie est nécessaire, l'os vertébral est découpé en forme de V (figures 15a et 15b) avec un angle correspondant à l'angle de lordose recherchée au final et donc correspondant à l'écartement angulaire des instruments de correction 10 l'un par rapport à l'autre (écartement selon l'angle α ). Pour se faire, le positionnement angulaire des instruments de correction 10 dans la position prédéfinie forme un guide pour les instruments de coupe de l'os de type ostéotome, les coupes de l'os vertébral en forme de V étant réalisées parallèles aux instruments de correction 10.

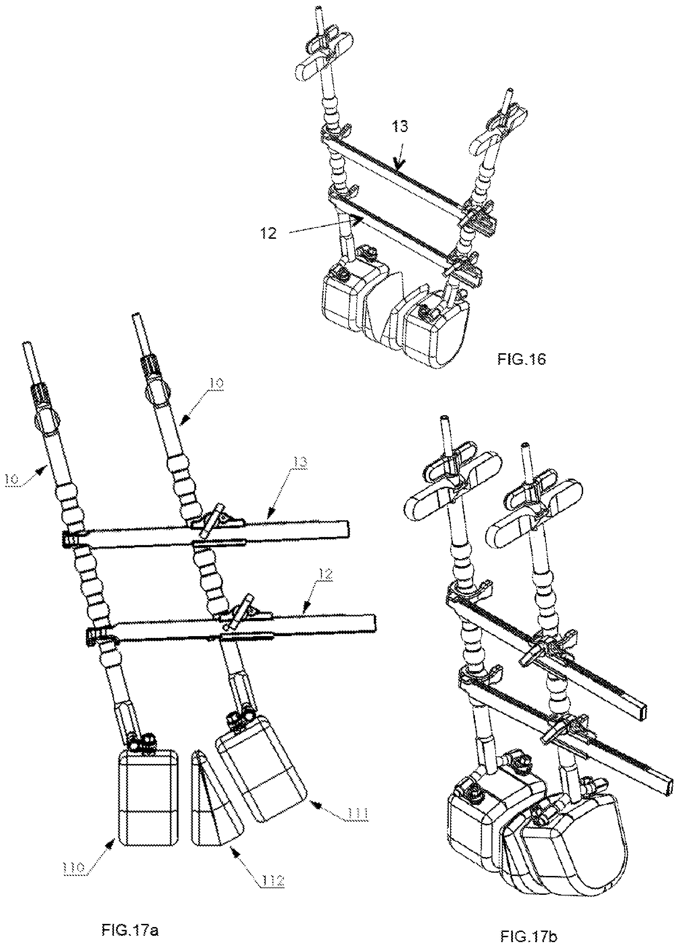

Afin d'assurer le contrôle de correction de compression/distraction des vertèbres instrumentées entre elles, un second instrument de manœuvre 13 peut être mis en place entre les deux instruments de correction 10 (figure 16).

Une fois les instruments de manœuvre en place on procède ensuite à la correction des vertèbres. Pour ce faire, on agit sur les instruments de correction 10 jusqu'à les placer parallèles entre eux (figures 17a et 17b), le positionnement parallèle final des instruments de correction 10 correspondant à la correction optimale de l'angle sagittale, les deux faces de la forme en V créées par l'ostéotomie éventuellement réalisée étant alors également parallèles, voire colinéaires.

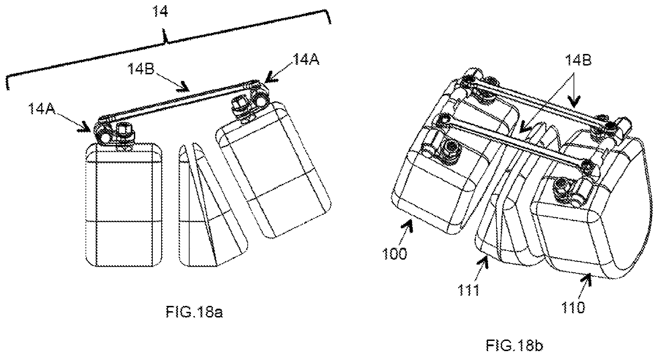

Une fois la correction effectuée, il ne reste plus qu'à lier les ensembles de fixation triangulaire entre eux afin de figer leur position avant le démontage des instruments de correction 10. Cette liaison peut être réalisée au moyen par exemple d'un système de fixation 14 longitudinal composé de crochets 14A et plaques 14B (figures 18a et 18b).

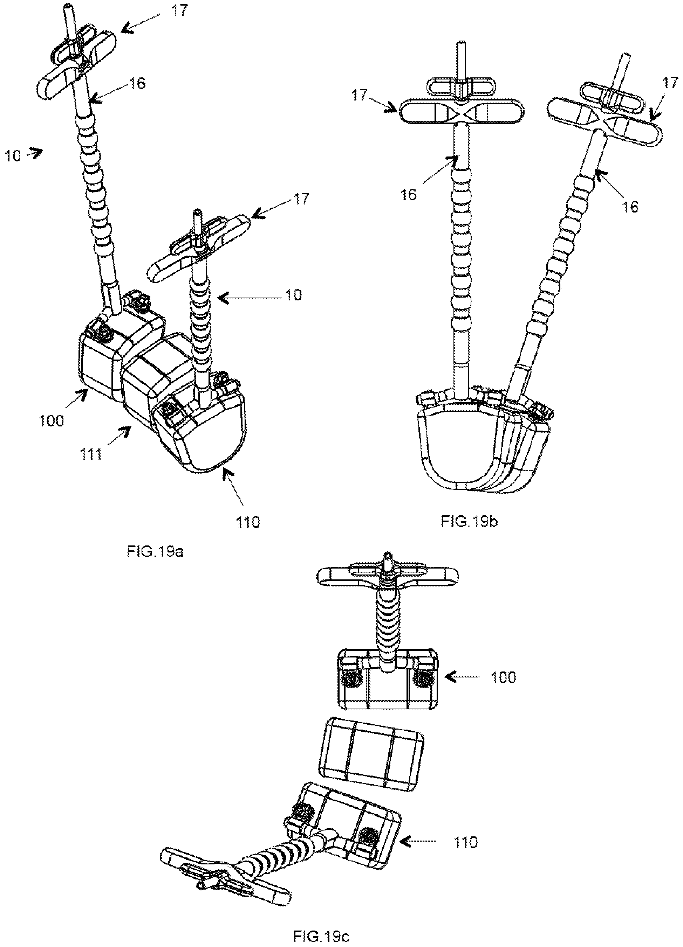

Les figures précédentes montrent des vertèbres nécessitant un redressement uniquement dans le plan sagittal. Il est bien entendu évident que le système de redressement selon l'invention ne se limite pas à ce type de correction, ce dernier étant adapté pour permettre des corrections dans les 3 dimensions. Les figures 19a à 19c montrent un exemple de vertèbres nécessitant une telle correction. Afin de faciliter la compréhension du mouvement des vertèbres réalisé lors de leur redressement à l'aide du système de redressement selon l'invention, les instruments de manœuvre n'ont pas été représentés.

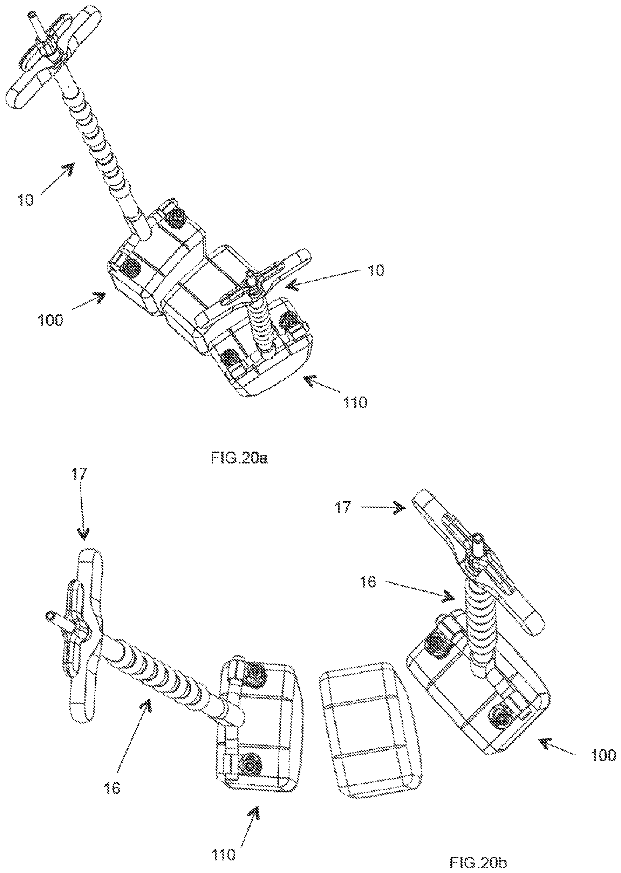

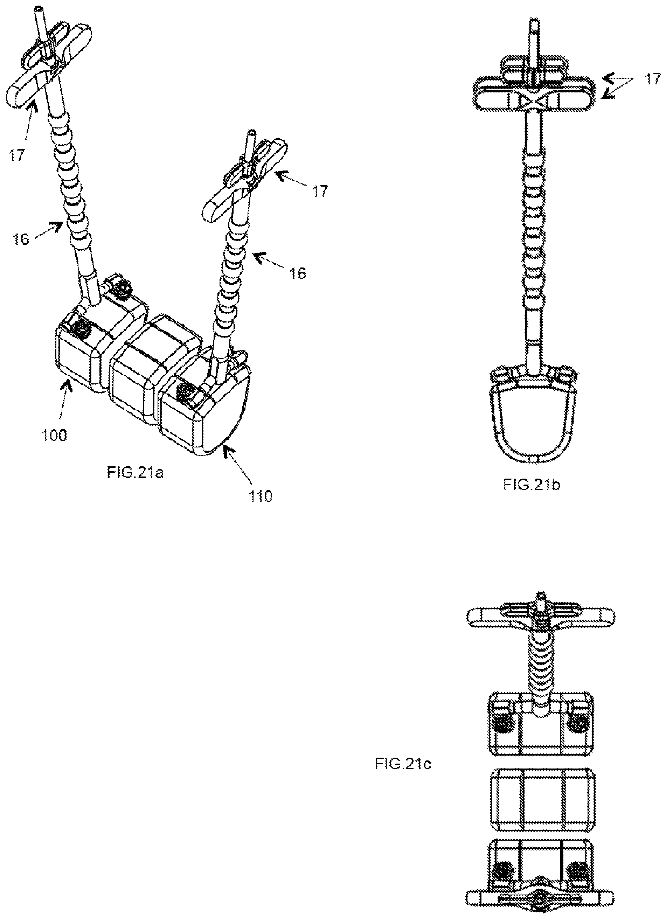

Ainsi, après avoir positionné les instruments de correction 10 de chaque ensemble de redressement l'un par rapport à l'autre selon l'angle α sagittal correspondant à la correction angulaire postéro-latéral visée, il est procédé à une correction de rotation transverse de façon à ramener les axes 16 des instruments de correction 10 dans un même plan, comme illustré sur les figures 20a et 20b. Il est procédé ensuite à une correction du déplacement coronal en positionnant les poignées 17 des instruments de correction 10 parallèles entre elles, comme illustré sur les figures 21 à 21c. Il est ensuite procédé à la correction sagittale des vertèbres en ramenant, comme dans l'exemple de redressement de vertèbres précédent, les axes 16 des instruments de correction 10 parallèles entre eux (figure 17a).

Les mouvements (rotation transverse, déplacement coronal et déplacement sagittal) appliqués aux vertèbres ont été décrits de manière séquentielle. Il est bien entendu évident que l'ensemble de ces mouvements peut être réalisés en un seul geste. L'instrument de mesure 15 décrit dans la phase initiale de positionnement angulaire des instruments de correction peut être également utilisé en cours de toutes les étapes chirurgicales décrites afin de contrôler et mesurer la progression de la correction.

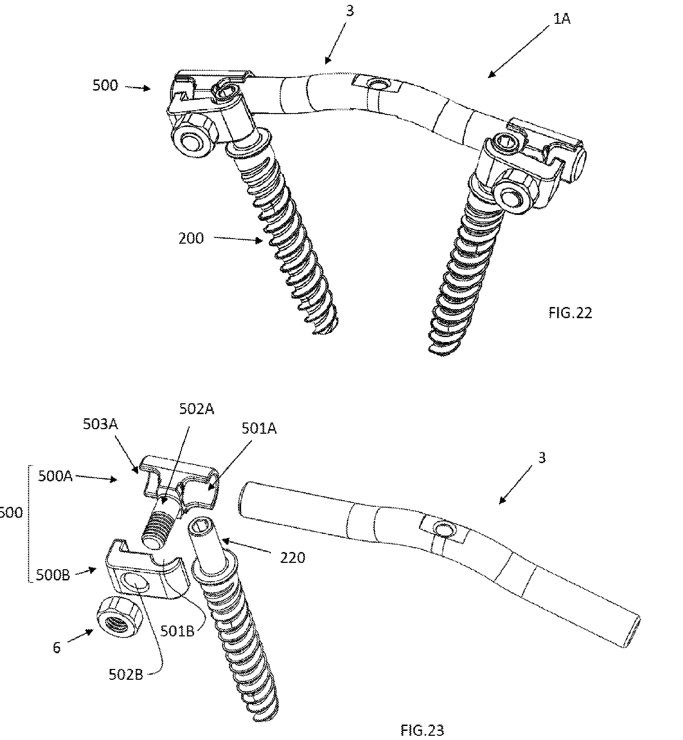

Les figures 22 et 23 représentent une variante de réalisation d'un ensemble de redressement 1A de vertèbres. La mise en œuvre de l'ensemble reste similaire avec celle précédemment décrite. L'ensemble de redressement de cette variante diffère cependant de celui précédemment décrit au niveau de l'arrangement des connecteurs 500 ainsi que des moyens d'ancrage 200.

Dans cette variante, le connecteur 500 est formé de deux pièces distinctes 500A, 500B,

L'une des pièces 500A forme un manchon destiné à recevoir la barre de triangulation 3. Le manchon est pourvu à chacune de ses deux extrémités, d'une encoche 501A, 503A. Il est pourvu sur sa face externe d'un axe fileté 502A. Les encoches sont disposées en regard l'une de l'autre, de part et d'autre de l'axe fileté.

L'autre pièce 500B comporte une gorge 501B arrangée pour recevoir la partie supérieure 220 du moyen d'ancrage osseux. Elle comporte un trou 502B pour le passage de l'axe fileté 502A. La gorge 501B est ménagée pour être disposée en vis-à-vis de l'une des encoches 501A. Ainsi, lorsque les deux pièces sont assemblées par vissage de l'écrou de serrage 6 sur l'axe filetée 502A qui s'étend au travers du trou de passage 502B, la partie supérieure 220 du moyen d'ancrage osseux et la barre de triangulation 3 sont serrées par pincement l'une contre l'autre, les deux pièces jouant le rôle d'étau.

Comme on le comprend, l'arrangement manchon/ tige filetée/partie supérieure du moyen d'ancrage osseux est tel qu'il permet un mouvement multiaxial de la barre de triangulation avant serrage complet de l'écrou, permettant ainsi le réglage de la position angulaire de l'instrument de correction monté sur la barre de triangulation (instrument non représenté).

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Ensemble de redressement (1, 1A) de vertèbres comprenant :
- deux moyens d'ancrage osseux (2, 200) destinés à être implantés sur une même vertèbre,
- une structure de pontage des moyens d'ancrage osseux entre eux, ladite structure de pontage, implantable, comprenant une barre de triangulation (3) et des moyens de connexion (4) permettant de raccorder la barre de triangulation (3) à chaque moyen d'ancrage osseux (2, 200),
- un instrument de correction (10) monté sur la barre de triangulation,
- des moyens de blocage de la barre de triangulation (3) sur les moyens d'ancrage osseux par l'intermédiaire des moyens de connexion, la barre de triangulation formant ainsi avec les moyens d'ancrage osseux un ensemble rigide de forme triangulaire, et
- des moyens de réglage de la position angulaire de l'instrument de correction (10) par rapport à la vertèbre selon un angle déterminé,
l'ensemble de redressement (1, 1A) de vertèbres étant **caractérisé en ce que** les moyens de connexion (4) comprennent deux connecteurs (5) comprenant chacun une première zone de passage définissant un axe longitudinal de passage pour la barre de triangulation (3) et une deuxième zone de passage arrangée pour recevoir l'un des moyens d'ancrage osseux, ladite deuxième zone de passage présentant une forme oblongue orientée parallèlement à l'axe longitudinal de passage de la première zone de passage, les connecteurs constituant les moyens de réglage de la position angulaire de l'instrument de correction (10).

2. Ensemble de redressement (1, 1A) de vertèbres selon la revendication 1, caractérisé en ce les moyens de connexion comportent les moyens de réglage de la position angulaire de l'instrument de correction (10).

3. Ensemble de redressement (1, 1A) de vertèbres selon la revendication 2, **caractérisé en ce que** la barre de triangulation (3) comporte une zone d'accroche (33) de l'instrument de correction (10) arrangée pour assurer une fixation solidaire en rotation et en translation de l'instrument de correction (10) sur la barre de triangulation.

4. Ensemble de redressement (1, 1A) de vertèbres selon la revendication 3, **caractérisé en ce que** la barre de triangulation (3) comporte un méplat formant la zone d'accroche (33).

5. Ensemble de redressement (1, 1A) de vertèbres selon la revendication 3 ou 4, **caractérisé en ce que** la zone d'accroche (33) est centrale.

6. Ensemble de redressement (1) de vertèbres selon l'une quelconque des revendications précédentes, **caractérisé en ce que** la première zone de passage est conformée pour permettre le coulissement et la rotation de la barre de triangulation (3) en son sein avant son blocage sur les connecteurs (5).

7. Ensemble de redressement (1) de vertèbres selon l'une quelconque des revendications précédentes, **caractérisé en ce que** chaque connecteur (5) se présente sous la forme d'une pince comprenant un partie ayant une section en C prolongée par deux bras comportant respectivement un orifice (523, 524), les orifices de chaque bras étant disposés en vis-à-vis l'un de l'autre pour définir la deuxième zone de passage tandis que la section en C délimite la première zone de passage.

8. Ensemble de redressement (1) de vertèbres selon la revendication 7, **caractérisé en ce que** les orifices (523, 524) sont arrangés pour présenter un décalage axial l'un par rapport à l'autre.

9. Ensemble de redressement (1, 1A) de vertèbres selon l'une quelconque des revendications précédentes, **caractérisé en ce que** chaque moyen d'ancrage osseux (2) comprend une tête de vis (20) fixe prolongée de part et d'autre par une partie filetée (21) inférieure pour l'implantation dans la vertèbre et une partie filetée (22) supérieure sur laquelle un écrou de serrage (6) formant les moyens de blocage est destinée à être vissé.

10. Ensemble de redressement (1, 1A) de vertèbres selon l'une quelconque des revendications précédentes, **caractérisé en ce que** la barre de triangulation (3) est droite ou cintrée.

11. Système d'ostéosynthèse rachidienne comprenant au moins deux ensembles de redressement de vertèbres selon l'une quelconque des revendications précédentes, lesdits ensembles (1, 1A) étant destinés à être implantés sur deux vertèbres distinctes et à être reliés entre eux.

12. Système d'ostéosynthèse rachidienne selon la revendication 11, **caractérisé en ce qu'**il comporte au moins un instrument de manœuvre reliant les instruments de correction (10) de chacun des ensembles de triangulation entre eux.

## Patentansprüche

1. Anordnung zur Aufrichtung (1, 1A) von Wirbeln, umfassend:
- zwei Knochenverankerungsmittel (2, 200), die dazu bestimmt sind, auf ein und demselben Wirbel implantiert zu sein,
- eine Struktur zur Überbrückung der Knochenverankerungsmittel untereinander, wobei die implantierbare Überbrückungsstruktur eine Triangulationsstange (3) und Verbindungsmittel (4) umfasst, die es ermöglichen, die Triangulationsstange (3) mit jedem Knochenverankerungsmittel (2, 200) zu verbinden,
- ein Korrekturinstrument (10), das an der Triangulationsstange angebracht ist,
- Mittel zum Arretieren der Triangulationsstange (3) an den Knochenverankerungsmitteln mittels der Verbindungsmittel, wobei die Triangulationsstange somit mit den Knochenverankerungsmitteln eine starre dreieckige Anordnung bildet, und
- Mittel zum Einstellen der Winkelposition des Korrekturinstruments (10) in Bezug auf den Wirbel gemäß einem bestimmten Winkel,
wobei die Anordnung zur Aufrichtung (1, 1A) von Wirbeln **dadurch gekennzeichnet ist, dass** die Verbindungsmittel (4) zwei Verbinder (5) umfassen, die jeweils einen ersten Durchgangsbereich, der eine Längsdurchgangsachse für die Triangulationsstange (3) definiert, und einen zweiten Durchgangsbereich umfassen, der eingerichtet ist, um eines der Knochenverankerungsmittel zu empfangen, wobei der zweite Durchgangsbereich eine längliche Form aufweist, die parallel zur Längsdurchgangsachse des ersten Durchgangsbereichs ausgerichtet ist, wobei die Verbinder die Mittel zum Einstellen der Winkelposition des Korrekturinstruments (10) bilden.

2. Anordnung zur Aufrichtung (1, 1A) von Wirbeln nach Anspruch 1, **dadurch gekennzeichnet, dass** die Verbindungsmittel die Mittel zum Einstellen der Winkelposition des Korrekturinstruments (10) umfassen.

3. Anordnung zur Aufrichtung (1, 1A) von Wirbeln nach Anspruch 2, **dadurch gekennzeichnet, dass** die Triangulationsstange (3) einen Befestigungsbereich (33) für das Korrekturinstrument (10) aufweist, der eingerichtet ist, um eine rotations- und translationsfeste Befestigung des Korrekturinstruments (10) an der Triangulationsstange zu gewährleisten.

4. Anordnung zur Aufrichtung (1, 1A) von Wirbeln nach Anspruch 3, **dadurch gekennzeichnet, dass** die Triangulationsstange (3) eine Abflachung aufweist, die den Befestigungsbereich (33) bildet.

5. Anordnung zur Aufrichtung (1, 1A) von Wirbeln nach Anspruch 3 oder 4, **dadurch gekennzeichnet, dass** der Befestigungsbereich (33) zentral ist.

6. Anordnung zur Aufrichtung (1) von Wirbeln nach einem der vorstehenden Ansprüche, **dadurch gekennzeichnet, dass** die erste Durchgangsbereich ausgebildet ist, um das Gleiten und die Rotation der Triangulationsstange (3) darin vor ihrem Arretieren an den Verbindern (5) zu gestatten.

7. Anordnung zur Aufrichtung (1) von Wirbeln nach einem der vorstehenden Ansprüche, **dadurch gekennzeichnet, dass** jeder Verbinder (5) in Form einer Klammer vorliegt, die einen Teil mit einem C-förmigen Querschnitt umfasst, der durch zwei Arme verlängert wird, die jeweils eine Öffnung (523, 524) aufweisen, wobei die Öffnungen jedes Arms einander gegenüberliegend angeordnet sind, um den zweiten Durchgangsbereich zu definieren, während der C-förmige Querschnitt den ersten Durchgangsbereich begrenzt.

8. Anordnung zur Aufrichtung (1) von Wirbeln nach Anspruch 7, **dadurch gekennzeichnet, dass** die Öffnungen (523, 524) so eingerichtet sind, dass sie einen axialen Versatz zueinander aufweisen.

9. Anordnung zur Aufrichtung (1, 1A) von Wirbeln nach einem der vorstehenden Ansprüche, **dadurch gekennzeichnet, dass** jedes Knochenverankerungsmittel (2) einen festen Schraubenkopf (20) umfasst, der auf beiden Seiten durch einen unteren Gewindeabschnitt (21) zur Implantation in den Wirbel und einen oberen Gewindeabschnitt (22) verlängert ist, auf den eine die Arretierungsmittel bildende Spannmutter (6) bestimmt ist, geschraubt zu werden.

10. Anordnung zur Aufrichtung (1, 1A) von Wirbeln nach einem der vorstehenden Ansprüche, **dadurch gekennzeichnet, dass** die Triangulationsstange (3) gerade oder gebogen ist.

11. Wirbelsäulen-Osteosynthesesystem, das mindestens zwei Anordnungen zur Aufrichtung von Wirbeln nach einem der vorstehenden Ansprüche umfasst, wobei die Anordnungen (1, 1A) dazu bestimmt sind, auf zwei verschiedenen Wirbeln implantiert und miteinander verbunden zu sein.

12. Wirbelsäulen-Osteosynthesesystem nach Anspruch 11, **dadurch gekennzeichnet, dass** es mindestens ein Bedienungsinstrument umfasst, das die Korrekturinstrumente (10) jeder der Triangulationsanordnungen miteinander verbindet.

## Claims

1. An assembly for rectifying (1, 1A) vertebrae, comprising:
- two bone anchoring means (2, 200) intended to be implanted on a same vertebra,
- a structure for bridging the bone anchoring means to one another, said bridging structure, which is implantable, comprising a triangulation bar (3) and connection means (4) for connecting the triangulation bar (3) to each bone anchoring means (2, 200),
- a correction instrument (10) mounted on the triangulation bar,
- means for locking the triangulation bar (3) on the bone anchoring means via the connection means, the triangulation bar thus forming, with the bone anchoring means, a rigid triangular assembly, and
- means for adjusting the angular position of the correction instrument (10) relative to the vertebra at a determined angle,
the assembly for rectifying (1, 1A) being **characterized in that** the connection means (4) comprise two connectors (5) each comprising a first passage area defining a longitudinal passage axis for the triangulation bar (3) and a second passage area arranged to receive one of the bone anchoring means, said second passage area having an oblong form oriented parallel to the longitudinal passage axis of the first passage area, the connectors forming the means for adjusting the angular position of the correction instrument (10).

2. The assembly for rectifying (1, 1A) vertebrae according to claim 1, **characterized in that** the connection means comprise means for adjusting the angular position of the correction instrument (10).

3. The assembly for rectifying (1, 1A) vertebrae according to claim 2, **characterized in that** the triangulation bar (3) comprises an attachment area (33) for the correction instrument (10), arranged to provide secure attachment, in rotation and in translation, of the correction instrument (10) on the triangulation bar.

4. The assembly for rectifying (1, 1A) vertebrae according to claim 3, **characterized in that** the triangulation bar (3) comprises a flattened portion forming the attachment area (33).

5. The assembly for rectifying (1, 1A) vertebrae according to claim 3 or 4, **characterized in that** the attachment area (33) is central.

6. The assembly for rectifying (1) vertebrae according to any one of the preceding claims, **characterized in that** the first passage area is shaped to enable the sliding and the rotation of the triangulation bar (3) within itself before it is locked on the connectors (5).

7. The assembly for rectifying (1) vertebrae according to any one of the preceding claims, **characterized in that** each connector (5) is in the form of a clip comprising a part having a C-shaped section extended by two arms comprising respectively an orifice (523, 524), the orifices of each arm being arranged facing one another in order to define the second passage area, while the C-shaped section delimits the first passage area.

8. The assembly for rectifying (1) vertebrae according to claim 7, **characterized in that** the orifices (523, 524) are arranged to have an axial offset relative to one another.

9. The assembly for rectifying (1, 1A) vertebrae according to any one of the preceding claims, **characterized in that** each bone anchoring means (2) comprises a fixed screw head (20) extended on either side by a lower threaded part (21) for implantation in the vertebra and an upper threaded part (22) onto which a retaining nut (6), forming the locking means, is intended to be screwed.

10. The assembly for rectifying (1, 1A) vertebrae according to any one of the preceding claims, **characterized in that** the triangulation bar (3) is straight or curved.

11. A system for spinal osteosynthesis comprising at least two assemblies for rectifying vertebrae according to any one of the preceding claims, said assemblies (1, 1A) being intended to be implanted on two discrete vertebrae and to be connected to one another.

12. The system for spinal osteosynthesis according to claim 11, **characterized in that** it comprises at least one maneuvering instrument connecting the correction instruments (10) of each of the triangulation assemblies to one another.
